# EUROPEAN PATENT SPECIFICATION

(11) **EP 1 200 040 B1**
(45) Date of publication and mention of the grant of the patent: **12.04.2006**
(21) Application number: 00947695.3
(22) Date of filing: 14.07.2000
(51) Int. Cl.: A61K 8/03, A61K 8/23, A61K 8/55, A61K 8/34, A61K 8/64, A61K 8/67, A61K 8/68, A61K 8/73, A61K 8/97, A61Q 19/00, A61Q 19/02, A61Q 19/08

(54) **TWO-COMPONENT COMPOSITION FOR COSMETIC OR PHARMACEUTICAL USE**
ZWEIKOMPONENTENZUSAMMENSETZUNGEN ZUR KOSMETISCHEN ODER PHARMAZEUTISCHEN VERWENDUNG
COMPOSITION BI-COMPOSANT D'USAGE COSMETIQUE OU PHARMACEUTIQUE

(30) Priority: 16.07.1999 BR 9902972
(43) Date of publication of application: 02.05.2002
(73) Proprietor: Industria e Comercio de Comesticos Natura Ltda, CEP-06850 Itapecerica da Serra, SP (BR)
(72) Inventor: ALCANTARA MARTINS ZUCCHETTI, Roberto, Sao Paulo, SP (BR); VILLA NOVA SILVA, Luciana, Sao Paulo, SP (BR); CHITARRA SOUZA, Simoni, Sao Paulo, SP (BR); FANAN, Simone, Campinas, SP (BR); GESZTESI, Jean-Luc, Sao Paulo, SP (BR); MARTINS MATHEUS, Luiz Gustavo, Sao Caetano do Sul, SP (BR); POMMEZ, Philippe Joseph, Sao Paulo, SP (BR)
(74) Representative: Scott, Susan Margaret
(86) International application number: PCT/BR2000/000077
(87) International publication number: WO 2001/005357

(56) References cited:
- WO-A-99/33439
- US-A- 5 094 783
- US-A- 5 626 883
- US-A- 5 804 168
- US-A- 5 902 591

## Description

### Field of the invention

The present invention rotates to an aqueous composition especially improved for cosmetic and pharmaceutical compositions, comprising antioxidant compounds and other beneficial compounds for the skin as moistufizers and immunomodulators.

### Prior Art

Cosmetic and pharmaceutical compositions including antioxidants are very well known for improving the skin aspect, e.g., preventing marks from showing on the skin, such as wrinkles, flaccidity, spots, or to treat mild problems such as irritations and other mild diseases.

In this kind of composition, antioxidant compounds are commonly used, such as levogyrous ascorbic acid (LAA), popularly known as "Vitamin C" and proanthocyanidins (OPC) because, among other characteristics, they act against the free radicals which accelerate the aging process and the cellular degeneration.

Said compositions lack, howewer, the supplying of a substantial improvement In the general quality of the skin, because they normally approach one or another problem singly. In addition, if on the one hand antioxidants bring improvement benefits for the skin or help to the celts health, on the other hand they can cause sensitivities to certain people.

When sensitivities occur, they can be attributed to the nature of certain antioxidants or to the concentrations required to obtain the desired benefits, which can vary from 5 to 10% or even 20% by weight. Lower antioxidants concentrations might not be potentially irritant, however, the effects they produce are below what would be desirable.

Still in that respect, compositions comprising antioxidants derivatives are very used. Only as an example, LAA esters can be used instead ascorbic acid in its molecular form (LAA). This kind of composition gives rise, until nowadays, to a great discussion among scientists, as for its effectiveness.

One fact that reinforces the theory of a better effectiveness of the antioxidants in their original form is the quantity of studies and publications related to the stabilization of such compounds. Examples of these studies are described in Brazilian patent applications Pl 9704418-0 and Pl 9704728-7 and in the prior art references mentioned therein, all of them dedicated to the LAA stabilization.

The OPC's, in their turn, are known by those skilled in the art from many already published works. One of these relates to the patent granted in the United States under number US 4,698,360, to Masquelier. Jacque.

In that patent anti-free radicals effects provided by such compounds are discussed, with therapeutic indications, including by oral, intravenous and topic route.

Compounds exclusively comprising OPC can bring beneficial effects to the user, however, they show a limited action and benefits scope.

In a further evolution, the patent granted in the United States to Lemer. Sheldom under number US 5.470,874 describes compositions comprising as main actives associated OPC's as a mixture to the ascorbic acid in its molecular form and derivatives as the ascorbyl palmitate.

As it can be noted from this last document mentioned, the composition taught therein to obtain a set of benefits for the skin is extremely complex, having always more than ten ingredients.

In addition to the composition complexity, the benefit scope provided by such composition is limited. Although the number of benefits counted is larger than the one described in US 4,698.360, these could bring some disadvantages.

A first disadvantage is a result of the high LAA contents used, always above 10%, sometimes reaching 25%, which cause a greater skin exfoliation and an uncomfortable sensation (buming), sometimes causing a contact allergy or even making the skin more sensitive to inflammations.

In addition, these high contents provide a very acid pH that causes a lower effectiveness in the complexing activity of the preservative Included therein, namely EDTA, which leads to a faster degradation of the LAA.

Summarizing, the aggressive condition provided by the combination of compounds disclosed in the above patent becomes damaging also because it is free from any present or associated entity capable of reducing such aggressiveness in such a way to supply a comfortable product for the user.

Therefore, it is an objective of the present Invention to provide a composition for cosmetic or pharmaceutical use which supplles a wide range of benefits, and white in use, provides to the user a solution of commitment between the expected effectiveness, due to the presence of antioxidants, and the comfort for the user, with superior results when compared with the known compositions as far as improvement of skin quality are concerned.

### Summary of the Invention

The present invention relates to a composition for cosmetic or pharmaceutical use characterized in that it comprises a first phase comprising, in an aqueous medium, ascorbic acid as an antioxidant compound present at 1 to 30% by weight, and a second phase which comprises 0.5 to 3.0% of ceramides as moisturizer and 0.5 to 3.0% of a betaglycane immunomodulator, the proportion between said first and second phases being in the range of 6:1 to 14:1.

### Detailed Description of the Invention

It was surprisingly found by the present inventors that a composition as defined above provides a wide range of benefits and, while in use, provides to the user both effectiveness, in view of the presence of antioxidant, and the comfort for the user, i.e., low potential of irritation, with superior results when compared with compositions known from the prior art as far as improvement of skin quality is concerned.

Antioxidants should be understood as compounds or mixture of compounds which have properties against the free radicals present in the body, especially in the skin.

Moisturizing agents useful for the present invention are compounds or mixtures of compounds able to restructure the skin barrier.

Immunomodulators are understood as any compound or mixture of compounds able to reinforce the skin immunological system.

According to a preferred embodiment of the present invention, the composition for cosmetic or pharmaceutical use comprises two different phases, which, due to their nature, can be conveniently packed in only one dispenser. Each phase, however, must be present in sealed compartments within said dispenser, which prevent contact between the phases prior to the moment of use, when they are simultaneously dispensed from said dispenser, e.g., due to compatibility between both of them, which could lead to the unstableness or degradation of the compounds as time goes by. An example of this kind of package is described in patent document WO 97/27841.

According to the above embodiment of the invention, the composition comprises in a first dispenser compartment, a first phase containing an aqueous composition comprising ascorbic acid, present at a concentration from 1 to 30% and, in a second phase, ceramides as moisturizer, from 0.5 to 3.0%, and the sodic betaglycan carboxyl immunomodulator, also known as betaglycane, present in a content from 0.5 to 3.0%, the application proportion between said first and second phases being from 6:1 to 14:1. All percentages set forth above are in weight relative to the total weight of the composition of each phase.

In a preferred embodiment of the present invention, such composition comprises in a first dispenser compartment, a first phase in which an aqueous composition is present, comprising is ascorbic acid, present at a percentage from 1 to 20%, and OPC's present from about 0.001 to 2.2% and, in a second phase, ceramides, preferably ceramides contained in a liquid crystal emulsion, also called lamellar ceramide, in a content from 0.5 to 3.0%, associated to the sodic betaglycan carboxyl immunomodulator, also known as betaglycane, present in a content from 0.5 to 3.0%, the application proportion between said first and second phases being from 6:1 to 14:1, preferably, between 12:1 to 8:1. All the percentages mentioned above are in weight relative to the total weight of the composition of each phase.

It was found that the use of compositions as described above improves strength and natural protection of the skin against external aggressions due to the association of the restructuring effect of the skin lipidic barrier by the lamellar ceramides with the reinforcement effect of the skin immunological system provided by the betaglycanes.

In an even more preferred embodiment of the invention, the composition comprises a first phase in a first dispenser compartment, where an aqueous composition is present comprising ascorbic acid present at a percentage from 1 to 20%, preferably between 5 and 18%, and OPC's, present from about 0.001 to 2.2%, preferably between 0.01 and 1.7% and, In a second phase, ceramides, preferably ceramides contained in a liquid crystal emulsion, also called lamellar ceramide, in a range from 0.5 to 3.0%, preferably

The illustrative examples and tests presented below will better describe the present invention. Nevertheless, the illustrated data and procedures relate merely to some embodiment of the present invention and should not be considered as restrictive of its scope.

### Example I:

A composition has been prepared comprising a first phase and a second phase, which between 1.5 to 2.5%, associated to sodic betaglycan carboxyl the immunomodulator, also known as betaglycane, which is present in a content from 0.5 to 3.0%, preferably between 1.5 to 2.5%, the application proportion between said first and second phases being from 6:1 to 14:1, preferably, between 12:1 to 8:1, and most preferably, around 11:1. All the percentages above mentioned are in weight relative to the total weight of the composition of each phase.

It has been observed that an adequate proportion between the first phase and the second phase is at about 6:1 to 14:1, preferably between 12:1 to 8:1 and, most preferably, around 11:1. The association of the two phases recovers the skin vitality and imparts an improvement of the skin strength and natural protection.

In vitro studies carried out by the inventors showed that the effects obtained with the composition of the invention can be improved when the ascorbic acid is the tevogyrous ascorbic acid (LAA), present within a selected concentration range.

Several essays were carried out by the inventors with the objective of determining in which concentration range the levogyrous ascorbic acid (LAA) has antioxidant and pro-oxidant activity and the results showed that LAA pro-oxidant action occurs in a concentration range between 0.005% and 0.01% while the antioxidant action occurs in ranges between 0.0001% and 0.001% and between 1 and 10% in these essays, it was also observed that at concentrations between 0.1 and 0.4% preferably around 0.3% of OPC's associated to the LAA in an aqueous medium, the pro-oxidant effect of the LAA present in the percentage range where it acts as pro-oxidant is inhibited. This conclusion derives from the observation that, in this essay, the deoxyribose degradation decreases 78%.

Advantageously, said first phase contains the antioxidants in their molecular stable or original form (without degradation) but their salts and esters could also be used certainly leading to good results. In this last case where salts, esters or polymerized antioxidants are to be used, it is believed that the separation between the first and the second phase prior to use could. In some particular cases be unnecessary. In addition, other compounds can be easily added to the above described components without representing much difficulty for those skilled in the art. Just as an example, such compounds indude fragrances, thickeners, and moisteners or other moisturizers. comprise:

### First Phase

| Ingredient | % mass |
|---|---|
| water | About 75 |
| Propylene glycol | 18 |
| Methyl paraben | 0.2 |
| Propyl paraben | 0.1 |
| Glutathion | 0.1 |
| 1-hydroxyethylidene (1,1 diphosphonic) acid (Dequest®) | 0.15 |
| LAA | from 1 to 30 |
| CPC | 0.3 |
| Modified Xantham Gum | 1.4 |

### Second Phase

| Ingredient | % mass | |
|---|---|---|
| Water | 76.20 | Vehicle |
| Xantham Gum | 0.27 | Thickener |
| Methyl paraben (Nipagin) | 0.18 | Preservative |
| Propyl pafaben (Nipazol) | 0.09 | Preservative |
| Carbomer (Carbopol 141) | 0.27 | Thickener |
| Glycerin | 9.09 | Moistener |
| Essence | 0.54 | Fragrance |
| Lamellar ceramide | 2.12 | Skin barrier restructuration agent |
| Sodium Lauril lactilate | 9.09 | Disperser |
| Sodium carboxymethyl betaglycan (Betaglycane) | 2.13 | Stimulating active |

The above composition permits the simultaneous application of selected proportions of compounds as defined in the first phase and in the second phase, what brings a surprising synergy, providing a wide range of benefits to the user among the beneficial effects of this product, many of which are explained in the further detailed tests, the following properties of the composition on the skin are detected:
- improves its exuberance:

- assuages the aging marks of skin (wrinkles and flaccidity):

- improves the skin tint, assuaging the spots appearance;
- stimulates and protect the skin immunological system;
- improves and recover the skin lipidic barrier;
- reduces dark circles;
- improves the aspect of varicose legs;
- improves oral affections (aphthae).

The results of the tests showed below prove the perception of several benefits by users, as well as prove the clinical effectiveness of the product.

A panel has been composed, in a blind study, with 45 volunteers evaluated at 4 moments immediately after the first application: after one week of use; after one fortnight of use, and after 30 days of use of the product. The supplied product had selected proportions of the first phase to the second phase about 11:1 and had the composition described in Example 1 above. In all the analyzed moments, the product was evaluated by the consumer and by the doctor. The results of this evaluation are described in Tables I and II, respectively, where the expressed percentages represent the amount of users who noticed that the correspondent benefit has indeed occurred.

**Table 1: Evaluation of the product performance by the consumer**

| Attributes | After 1st Application (%) | After 7 days (%) | After 15 days (%) | After 30 days (%) |
|---|---|---|---|---|
| Protection | 8.9 | 86.7 | 86.7 | 93.3 |
| Skin lightening | 40.0 | 80.0 | 91.1 | 95.5 |
| More exuberance | 86.7 | 95.5 | 97.8 | 97.8 |
| Improvement of softness | 93.3 | 95.5 | 97.8 | 97.8. |
| Improvement of tensing action | 93.3 | 95.5 | 95.5 | 95.5 |
| Moisture Improvement | 93.3 | 97.8 | 97.8 | 97.8 |
| lmprovement of fine lines (fine wrinkles) Cared/Nourished | 15.5 | 55.5 | 64.5 | 68.9 |
| | 68.4 | 97.8 | 97.8 | 97.8 |

**Table 2. Evaluation of the product performance by the doctor**

| Attributes | After 1st Application (%) | After 7 days (%) | After 15 days (%) | After 30 days (%) |
|---|---|---|---|---|
| Skin lightening | 13.3 | 60.0 | 68.9 | 68.9 |
| Improvement of softness | 95.5 | 95.5 | 97.5 | 97.8 |
| Improvement of tensing action | 82.2 | 93.3 | 93.3 | 93.3 |
| Moisture improvement | 95.5 | 97.8 | 97.8 | 97.8 |
| Improvement of fine lines | 17.8 | 55.5 | 62.2 | 68.9 |

It was observed that the above mentioned results are achieved firstly due to the selection of the nature of the antioxidant compounds and of the moisturizing agents associated to immunomodulators, which, when in contact with skin, produce an advantageous synergetic effect. It is worth observing that in the group of users, all of them indistinctly noticed an improvement greater than 93% for seven out of eight benefits evaluated.

In addition, equally important, the proportion of availability of the first and second phases permitted to find a solution of commitment between the effective antioxidant action and the comfort to the user.

## Claims

1. Composition for cosmetic or pharmaceutical use **characterized in that** it comprises a first phase comprising, in an aqueous medium, ascorbic acid as an antioxidant compound present at 1 to 30% by weight, and a second phase which comprises 0.5 to 3.0% of ceramides as moisturizer and 0.5 to 3.0% of a betaglycane immunomodulator, the proportion between said first and second phases being in the range of 6:1 to 14:1.

2. Composition according to claim 1, **characterized in that** the first phase comprises ascorbic acid present at 1 to 10% by weight.

3. Composition according to claim 1, **characterized in that** the first phase comprises a mixture of antioxidants comprising ascorbic acid, present at 1 to 20%, and proantocyanidins (OPC's), present at 0.001 to 2.2%, wherein the second phase comprises 0.5 to 3.0% by weight of ceramides as moisturizer, preferably lamellar ceramides, and 0.5 to 3.0% of betaglycane immunomodulator, the proportion of use between said first and second phases being from 6:1 to 14:1, preferably between 12:1 to 8:1.

4. Composition according to claim 3, **characterized in that** the antioxidant compound of the first phase comprises 5 to 18% of ascorbic acid and 0.01 to 1.7% of OPC's and the second phase comprises 1.5 to 2.5% of lamellar ceramides as moisturizer, and 1.5 to 2.5% of betaglycane as the immunomodulator, the proportion between said first and second phases being from 12:1 to 8:1, preferably around 11:1.

5. Composition according to either claim 3 or claim 4, **characterized in that** the ascorbic acid is in the levogyrous molecular form, the OPC's are grape seed oligomers present at 0.1 to 0.4%, preferably around 0.3%, wherein said first and second phases are maintained separated prior to the moment of use.

6. Composition according to claim 5, **characterized in that** the first phase also includes from 15 to 19% of propylene glycol, from 0.01 to 1% of methyl paraben, from 0.05 to 1% of propyl paraben, from 0.05 to 0.5% of glutathion, from 0.1 to 0.5% of 1-hydroxyethylidene (1,1 diphosphonic) acid, the balance being water in enough quantity to complete 100% of the weight of such phase; wherein the second phase also includes compounds selected from xantham gum thickeners, carbomer and its mixtures, present at from 0.3 to 0.7%, selected methyl paraben, propyl paraben preservatives and mixtures thereof, present at 0.09 to 0.27%.

7. The use of a composition as claimed in any one of claims 1 to 6 as a cosmetic.

## Patentansprüche

1. Zusammensetzung zur kosmetischen oder pharmazeutischen Verwendung enthaltend eine erste Phase, die in einem wässrigen Medium Ascorbinsäure als Antioxidans in einer Menge von 1 bis 30 Gew.-% enthält, und eine zweite Phase, die 0,5 bis 3 % Ceramide als Feuchtigkeitscreme und 0,5 bis 3 % eines Betaglycan-Immunomodulators enthält, wobei das Verhältnis der ersten zur zweiten Phase im Bereich von 6:1 zu 14:1 liegt.

2. Zusammensetzung nach Anspruch 1,
**dadurch gekennzeichnet, dass** die erste Phase Ascorbinsäure in einer Menge von 1 bis 10 Gew.-% enthält.

3. Zusammensetzung nach Anspruch 1,
**dadurch gekennzeichnet, dass** die erste Phase eine Mischung von Antioxidantien enthaltend 1 bis 20 % Ascorbinsäure und 0,001 bis 2,2 % Proanthocyanidine (OPC's) enthält, wobei die zweite Phase 0,5 bis 3 Gew.-% Ceramide, insbesondere lamellare Ceramide, als Feuchtigkeitscreme und 0,5 bis 3 % Betaglycan-Immunomodulator enthält und bei der Verwendung das Verhältnis zwischen der ersten und der zweiten Phase im Bereich von 6:1 bis 14:1, bevorzugt zwischen 12:1 bis 8:1, liegt.

4. Zusammensetzung nach Anspruch 3,
**dadurch gekennzeichnet, dass** das Antioxidans der ersten Phase 5 bis 18 % Ascorbinsäure und 0,01 bis 1,7 % OPC's enthält und die zweite Phase 1,5 bis 2,5 % lamellare Ceramide als Feuchtigkeitscreme und 1,5 bis 2,5 % Betaglycan als Immunomodulator enthält, wobei das Verhältnis zwischen der ersten und der zweiten Phase im Bereich von 12:1 bis 8:1, bevorzugt bei etwa 11:1, liegt.

5. Zusammensetzung nach einem der Ansprüche 3 oder 4,
**dadurch gekennzeichnet, dass** die Ascorbinsäure in der lävogyr-molekularen Form vorliegt und die OPC's Traubensamen-Oligomere in einer Menge von 0,1 bis 0,4 %, bevorzugt etwa 0,3 % sind, wobei die erste und die zweite Phase vor dem Moment der Anwendung getrennt aufbewahrt werden.

6. Zusammensetzung nach Anspruch 5,
**dadurch gekennzeichnet, dass** die erste Phase zusätzlich 15 bis 19 % Propylenglykol, 0,01 bis 1 % Methylparaben, 0,05 bis 1 % Propylparaben, 0,05 bis 0,5 % Glutathion, 0,1 bis 0,5 % 1-Hydroxyethyliden (1,1-Diphosphon)-Säure enthält und als Ausgleich Wasser in einer ausreichenden Menge vorliegt, um 100 Gew.-% dieser Phase zu vervollständigen, wobei die zweite Phase zusätzlich Verbindungen ausgewählt aus Xanthan-Verdickungsmitteln, Carbomer und deren Mischungen in einer Menge von 0,3 bis 0,7 % sowie Methylparaben-, Propylparaben-Konservierungsmittel und Mischungen hiervon in einer Menge von 0,09 bis 0,27 % enthält.

7. Verwendung der Zusammensetzung nach einem der Ansprüche 1 bis 6 als Kosmetikum.

## Revendications

1. Composition destinée à une utilisation cosmétique ou pharmaceutique, **caractérisée en ce qu'**elle comprend une première phase comprenant, dans un milieu aqueux, de l'acide' ascorbique en tant que composé antioxydant présent à raison de 1 à 30 % en poids, et une seconde phase qui comprend de 0,5 à 3,0 % de céramides en tant qu'agent hydratant et de 0,5 à 3,0% d'un bêtaglycane immunomodulateur, la proportion entre lesdites première et seconde phases se situant dans la plage de 6:1 à 14:1.

2. Composition selon la revendication 1, **caractérisée en ce que** la première phase comprend de l'acide ascorbique présent à raison de 1 à 10 % en poids.

3. Composition selon la revendication 1, **caractérisée en ce que** la première phase comprend un mélange d'antioxydants comprenant de l'acide ascorbique, présent à raison de 1 à 20 %, et des proanthocyanidines (OPC) présentes à raison de 0,001 à 2,2 %, dans laquelle la seconde phase comprend de 0,5 à 3,0 % en poids de céramides en tant qu'agent hydratant, de préférences des céramides lamellaires, et de 0, 5 à 3,0 % de rétaglycane immunomodulateur, la proportion d'utilisation entre lesdites première et seconde phases allant de 6:1 à 14:1, de préférence de 12:1 à 8:1.

4. Composition selon la revendication 3, **caractérisée en ce que** le composé antioxydant de la première phase comprend de 5 à 18 % d'acide ascorbique et de 0,01 à 1,7 % d'OPC, et la seconde phase comprend de 1,5 à 2,5 % de céramides lamellaires en tant qu'agent hydratant et de 1,5 à 2,5 % de bêtaglycane en tant qu'immunomodulateur, la proportion entre lesdites première et seconde phases étant de 12:1 à 8:1, de préférence d'environ 11:1.

5. Composition selon l'une quelconque des revendications 3 ou 4, **caractérisée en ce que** l'acide ascorbique se trouve sous forme moléculaire lévogyre, les OPC sont des oligomères de pépins de raisin présents à raison de 0,1 à 0,4 %, de préférence d'environ 0,3 %, dans laquelle lesdites première et seconde phases sont maintenues séparées avant le moment de leur utilisation.

6. Composition selon la revendication 5, **caractérisée en ce que** la première phase comprend également de 15 à 19 % de propylène glycol, de 0,01 à 1 % de methyl paraben, de 0,05 à 1 % de propyl paraben, de 0,05 à 0,5 % de glutathion, de 0,1 à 0,5 % d'acide 1-hydroxyéthylidène (1,1 diphosphonique), le reste étant constitué d'eau en quantité suffisante pour atteindre 100 % du poids de cette phase ; dans laquelle la seconde phase comprend également des composés choisis parmi les épaississants de gomme de xanthane, un carbomer et leurs mélanges, présents à raison de 0,3 à 0,7 %, de conservateurs choisis à base de methyl paraben, de picopyl paraben et leurs mélanges, présents à raison de 0, 09 à 0,27 %.

7. Utilisation d'une composition selon l'une quelconque des revendications 1 à 6 en tant que produit cosmétique.
